(19) Europäisches Patentamt
European Patent Office
Office européen des brevets

(11) **EP 4 715 375 A1**

(12) **EUROPEAN PATENT APPLICATION**
published in accordance with Art. 153(4) EPC

(43) Date of publication:
**25.03.2026 Bulletin 2026/13**

(21) Application number: **24734935.0**

(22) Date of filing: **16.05.2024**

(51) International Patent Classification (IPC):
*G01N 21/952* (2006.01)    *G01N 21/954* (2006.01)
*B23K 31/12* (2006.01)      *B65B 51/22* (2006.01)
*G01N 1/28* (2006.01)       *G01N 1/32* (2006.01)
*G01N 1/34* (2006.01)       *G06T 7/00* (2017.01)

(52) Cooperative Patent Classification (CPC):
**G01N 21/952; G01N 21/954; G06T 7/0004;**
B23K 31/125; G01N 1/286; G01N 1/32; G01N 1/34;
G01N 17/006; G01N 33/2045; G01N 2001/2866;
G01N 2021/9544; G01N 2203/0066;
G06T 2207/30136

(86) International application number:
**PCT/ES2024/070297**

(87) International publication number:
**WO 2024/236214 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.05.2023 ES 202330376**

(71) Applicant: **Consejo Superior De Investigaciones
Científicas
(CSIC)
28006 Madrid (ES)**

(72) Inventors:
• **FERNÁNDEZ SERRANO, Ricardo
28040 Madrid (ES)**
• **PEÑA FERNÁNDEZ, Ma Carmen
28040 Madrid (ES)**

(74) Representative: **Cueto, Sénida
SP3 Patents S.L.
Los Madroños, 23
28891 Velilla de San Antonio (ES)**

(54) **SYSTEM FOR ASSESSING THE TOUGHNESS OF LONGITUDINAL HIGH FREQUENCY INDUCTION OR RESISTANCE WELDS IN STEEL TUBES AND ASSOCIATED METHOD**

(57) The present invention falls within the technical field of systems and methods for the evaluation of longitudinal welds in steel tubes, and more specifically of longitudinal welds made by high frequency induction welding (HFW) or electric resistance welding (ERW), and consists of a system for the evaluation of the toughness of longitudinal welds by high frequency induction or electric resistance welding in steel tubes and an associated method.

**FIG. 3**A

## Description

### OBJECT OF THE INVENTION

The present invention is framed in the technical field of systems and methods for the evaluation of longitudinal welds in steel pipes, and more specifically of longitudinal welds made by high frequency induction heating (HFW, High Frequency Welding) or by resistance welding (ERW, Electric Resistance Welding).

The object of the invention consists of a system for the evaluation of the toughness of longitudinal welds by high frequency induction or resistance welding in steel tubes and an associated method, where the associated method can be carried out in air or in the presence of gases which can embrittle the steel, such as hydrogen or mixtures of hydrogen with other gases.

### BACKGROUND OF THE INVENTION

Nowadays there are a multitude of pipes all over the world manufactured with different diameters and wall thicknesses by resistance welding or induction welding. These pipes are manufactured in different steel compositions (Grade B, X42, X52, X60, X70 and X80 in the gas transportation industry) and have application mainly for the transportation of low pressure fluids (typically<=200bar), such as natural gas, oil or water. In addition, these pipes are used in other applications such as foundation work, the automotive industry and for the protection of communication cables.

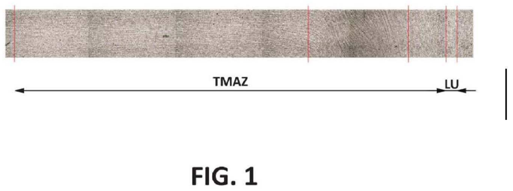

High-frequency induction or resistance welding is a complex process that is carried out without filler material or shielding gas and in which the welding operation is determined by a large number of parameters, some of which are interrelated. These parameters affect the weld quality and the microstructure of the different typical regions called: bond line (LU), thermo-mechanically affected zone (TMAZ) and thermally affected zone (HAZ), as shown in Figure 1.

The welding process, and in particular its adjustment, depends mainly on the chemical composition of the material used to manufacture the tube: the frequency of the induced electric field, the position of the inductor, the maximum temperature reached, the feed rate of the tube as it passes through the inductor or the resistor, the mechanical stresses applied on the material at the welding point and the angle (vee) at the point of contact of the plates.

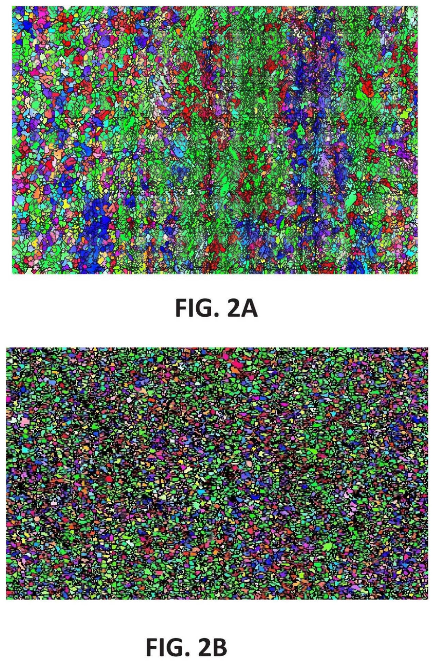

The high frequency induction welding process generates a microstructure around the bond line (LU) with very low toughness, i.e. low resistance to crack propagation. This low toughness is microstructurally justified by the clustering of grains with the same crystallographic orientation around the bond line (LU), and also by the presence of low toughness and high hardness phases such as retained austenite or martensite. Each color in Figure 2 represents a crystallographic orientation. Moreover, such a zone of inherent low toughness is especially damaging as it is perfectly aligned along the longitudinal direction of the tube. To avoid the low toughness of the tubes after the welding process, the welded tubes are subjected to a high temperature heat treatment called normalizing (or double normalizing), or quenching + tempering. Such treatments are usually effective in modifying, preferably to the point of practically eliminating, the detrimental microstructure around the bond line (LU) generated in the high frequency induction or resistance welding process. If such heat treatment is properly performed, brittle phases (e.g. martensite) and the clustering of grains with the same crystallographic orientation should disappear, but this is not always the case. Despite the use of single normalizing or even double normalizing post-weld treatments, low bond line (LU) toughness sometimes remains.

The evaluation of the integrity of high frequency induction or resistance welding on pipes is performed differently in the factory and when the pipe is in service. During the manufacturing process, the quality control of the pipe is performed by inspecting the process parameters used during welding (temperature, feed rate of the pipe through the inductor or resistor, stresses generated on the material at the welding point, vee angle, electrical characteristics of the induction process, etc). (Experimental Investigation of Temperature and Contact Pressure Influence on HFI Welded Joint Properties, Christian Egger, Martin Kroll, Kerstin Kern, Yannik Steimer, Michael Schreiner and Wolfgang Tillmann). In addition, a visual inspection of the weld is performed and in the highest quality level in the API 5L standard, PSL2, a series of destructive mechanical tests, including the impact test (Charpy test) are conducted to evaluate the toughness of the weld. Pipelines in service are periodically inspected to assess their integrity and detect the presence of cracks, corrosion damage and other defects that could jeopardize the installation. This inspection and study of the integrity of thepipelines is usually carried out by means of systems, called PIGS (Pipeline Integrity Gauges), which travel inside the pipe thanks to the pressure of the fluid being transported in the installation and detect defects by means of technologies such as magnetic flux leakage or ultrasounds. However, the use of these systems is limited to large diameter pipes and installations designed to allow the movement of the PIG through the installation, for example, avoiding sharp bends in its path. In any case, these systems cannot determine the value of pipe toughness in service. Toughness is the key aspect that differentiates the two quality levels of HFW/ERW pipes, described as PSL1 and PSL2 according to API 5L. PSL1 pipes must meet a number of requirements, such as the chemical composition of the steel, type of pipe end finish (plain, threaded or flared), etc. PSL2 pipes, of higher quality than PSL1, have mechanical requirements on yield strength, maximum stress and toughness (resilience) at the bond line (LU). Categorizing a pipe as PSL1 or PSL2 without mechanical testing remains a challenge for the low pressure fluid transportation industry such as in gas pipelines. Furthermore, despite the fact that numerous quality controls are applied to HFW and ERW pipes both in the factory and in service, hundreds of accidents associated with the lower bond line toughness with respect to the base material of the pipe occur annually. One of the reasons for this large number of accidents is that the evaluation of the bond line (LU) toughness of new pipes in the factory has not been properly performed until the last modification of the European standard ISO 3183 Oil and natural gas industries. Steel pipes for pipeline transportation systems (ISO 3183:2019). The determination of the toughness (resilience) of the bond line (LU) according to these standards is performed by Charpy tests at low temperature (maximum 0°C). The performance of these tests has several negative aspects such as destructive testing, cost and time required to properly machine the specimens and test time. In particular, the evaluation of the bond line (LU) toughness requires the specimens to be machined with a very high precision with respect to the bond line (LU), +/- 250$\mu$m around the bond line (LU) at most. This procedure has been included in the latest version of ISO 3183:2019 but although it allows evaluating the bond line (LU) toughness of HFW and ERW tubes, it is also a destructive evaluation procedure.

In recent years, promising new systems have been developed to evaluate in-service pipe toughness nondestructively by mechanical surface testing by performing controlled scratching or peeling processes on the pipe surface, as in "Nondestructive Evaluation for Yield Strength and Toughness of Steel Pipelines" S. D. Palkovic, K. Taniguchi, S. C. Bellemare: Massachusetts Materials Technologies, LLC, Cambridge, MA 2: Massachusetts Institute of Technology, Cambridge, MA. Conference: NACE 2018. At: Phoenix, AZ. These methods perform a friction-slip procedure and characterize the generated grooves or ligaments to respectively evaluate the hardness and toughness around the bond line (LU). However, this method is not purely experimental and relies on the use of data from finite element simulations. Furthermore, the above paper acknowledges that further testing and calibration is still needed to validate the method in real cases.

In addition, the current global decarbonization trend has led to the reuse of gas transportation infrastructures to transport hydrogen or mixtures of hydrogen and other gases (mainly natural gas). The presence of hydrogen in gas pipelines will reduce the toughness of components, mainly welds, including HFW/ERW. Such reduction in toughness is directly related to the presence of high hardness zones, strong microstructural gradients and compositional segregations (e.g. MnS). At the moment there is no system that allows evaluating the toughness of HFW/ERW welds in air, hydrogen or hydrogen/natural gas mixtures with other gases in a non-destructive way.

The system for the evaluation of the toughness of longitudinal welds by high-frequency induction or by resistance in steel tubes and the associated method solve all the above drawbacks.

## DESCRIPTION OF THE INVENTION

The present invention relates to a system for the evaluation of the toughness of longitudinal high frequency induction welds (HFW) or electric resistance welds (ERW) on steel pipes which provides a systematic and non-destructive evaluation of the bond line toughness from the microstructure characteristics around the bond line in the outer or inner zones of the pipe, on steel pipes that provides a systematic and non-destructive evaluation of bond line toughness from the characteristics of the microstructure around the bond line on the outside of the pipe in the area of the longitudinal weld made by high frequency induction heating or resistance heating without mechanical testing.

A system for evaluating the toughness of longitudinal high frequency induction or resistance welds in steel tubes comprises:

- image acquisition means configured to acquire, in use, at least a two-dimensional image of an outer or inner surface of the tube in an analysis region around a bond line (LU) of the longitudinal weld, wherein the two-dimensional image comprises a first dimension essentially perpendicular to the bond line of the weld and a second dimension essentially parallel to the bond line, and wherein the two-dimensional image comprises data associated with the microstructure of the tube in the analysis region;
- processing means configured to provide from the data associated with the microstructure of the tube in the analysis region, a greyscale profile with respect to the first dimension; and
- analysis means configured to analyse the greyscale profile and categorize the toughness of the tube in the analysis region around the bond line of the longitudinal weld.

In this way, by means of the at least one two-dimensional image it is possible to obtain the greyscale profile of the different zones of the tube microstructure around the bond line in the analysis region, where the analysis region preferably corresponds to a thermo-mechanically affected zone (TMAZ). More preferably, the analysis region is a 25x25mm region.

Thus, it is possible to categorize the bond line (LU) toughness from an image of the outer or inner surface of the tube, preferably considering the microstructural gradients in the zone and optionally the hardness of the zone under study or compositional segregations.

The toughness requirement to categorize a tube as PSL2 is based on the material having a homogeneous microstructure (including crystallographic orientation), around the bond line, and optionally on there being no zones of high hardness, nor significant compositional segregations (e.g. MnS). During the tube manufacturing process, microstructural gradients, including heat treatments, are generated in the analysis region.

The microstructure of the material around the bond line, for the steels used to manufacture the tubes that are welded by high frequency induction or resistance welding is mainly composed of ferrite and pearlite or bainite, although in some grades from X60 onwards it is also possible to find retained austenite and/or martensite. If the welding process or the subsequent quenching and tempering or normalizing heat treatments have not been carried out correctly, the toughness of the weld is not adequate and this low toughness is reflected in some aspects of the microstructure.

The system of the present invention provides a systematic and non-destructive evaluation of the thoughness of the bond line from the characteristics of the microstructure around the bond line in the outer or inner zones of the tube in the area of the longitudinal weld.

Optionally, the system further comprises means for coupling the image acquisition means to the tube. Preferably, the coupling means comprise a support comprising a lower edge having a curvature essentially equal to the curvature of the tube.

Optionally, the coupling means comprise anchoring means configured to attach the image acquisition means to the surface of the tube. Preferably, the anchoring means comprise magnetic or electromagnetic anchors.

Optionally, the system further comprises automatic grinding means configured, in use, to remove the outer or inner layers of the tube, preferably paint and rust, down to the metallic material.

Optionally, the system further comprises sanding means configured, in use, to sand the metallic material of the tube.

Optionally, the system further comprises polishing means configured, in use, to obtain a mirror-like glossy finish on the tube.

Optionally, the system further comprises chemical means configured, in use, to chemical etch the tube.

Optionally, the system further comprises cleaning means configured, in use, to wash away the film left on the tube by the chemical means and leave no marks in the analysis region.

Optionally, the image acquisition means are digital image acquisition means.

Optionally, the system further comprises a robotic module configured to move along the tube the surface cleaning means and/or the image acquisition means.

The invention also relates to a method for the evaluation of the toughness of longitudinal welds by high-frequency induction or resistance welding in steel tubes comprising:

- an image acquisition stage wherein at least one two-dimensional image of an outer or inner surface of the tube is acquired in an analysis region around a bond line (LU) of the longitudinal weld, wherein the acquisition stage is carried out such that the two-dimensional image comprises a first dimension essentially perpendicular to the bond line of the weld and a second dimension essentially parallel to the bond line, and wherein in the acquisition stage data associated with the microstructure of the tube in the analysis region are obtained from the two-dimensional image;
- a processing stage where a greyscale profile with respect to the first dimension is provided from the data associated with the microstructure of the tube in the analysis region; and
- an analysis stage where the greyscale profile is analysed and the toughness of the tube in the analysis region around the longitudinal weld bond line is categorized.

Optionally, the method further comprises a stage of evaluating the hardness of the area around the bond line (LU) from the obtained greyscale profile.

Optionally, the method further comprises a coupling stage to the tube prior to the image acquisition stage.

Optionally, the method further comprises an automatic roughing stage wherein the outer or inner layers of the tube are removed down to the metallic material.

Optionally, the method further comprises a sanding stage wherein the sanding of the metallic material of the tube is carried out.

Optionally, the method further comprises a stage of polishing the tube to obtain a mirror-like bright finish of the tube.

Optionally, the method further comprises a stage of chemical etching of the tube.

Optionally, the method further comprises a cleaning stage wherein the film left on the tube after the chemical etching stage is removed.

**DESCRIPTION OF THE DRAWINGS**

To complement the description being made and in order to help a better understanding of the characteristics of the invention, in accordance with a preferred example of practical realization of the same, there is attached as an integral part of said description, a set of drawings in which the following has been represented for illustrative and non-limiting purposes:

Figure 1.- Shows a view of the different regions of the tube around the bond line.

Figure 2a.- Shows a scanning electron microscopy image of the backscattered electron diffraction type applied to an analysis region around a bond line (LU) of the longitudinal weld for a low toughness X60 tube without normalizing treatment.

Figure 2b.- Shows a scanning electron microscopy image of the backscattered electron diffraction type applied to an analysis region around a bond line (LU) of the longitudinal weld for an X60 PSL2 pipe according to API 5L standard.

Figure 3a.- Shows a perspective view of the image acquisition means arranged in the coupling means of the system for the evaluation of the toughness of longitudinal welds by high frequency induction or by resistance in steel pipes of the present invention.

Figure 3b.- Shows a perspective view of the image acquisition means arranged in the coupling means of the system located inside the tube for the evaluation of the toughness of longitudinal welds by high frequency induction or by resistance in steel tubes of the present invention.

Figure 4.- Shows a schematic view of the system for the evaluation of the toughness of longitudinal welds by high frequency induction or resistance welding in steel tubes of the present invention.

Figure 5a.- Shows the microstructural zone around the bond line for a first master specimen (Master 1) without normalising treatment, with low toughness for the X60 steel tube of figure 2a.

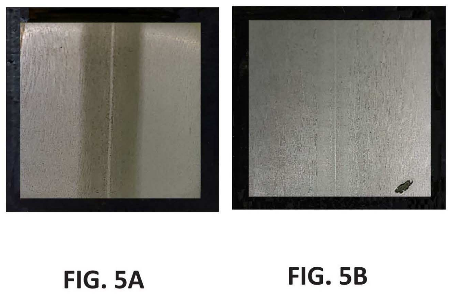

Figure 5a.- Shows the microstructural zone around the bond line for a sample with normalising treatment, high toughness AT for the X60 steel tube in Figure 2b.

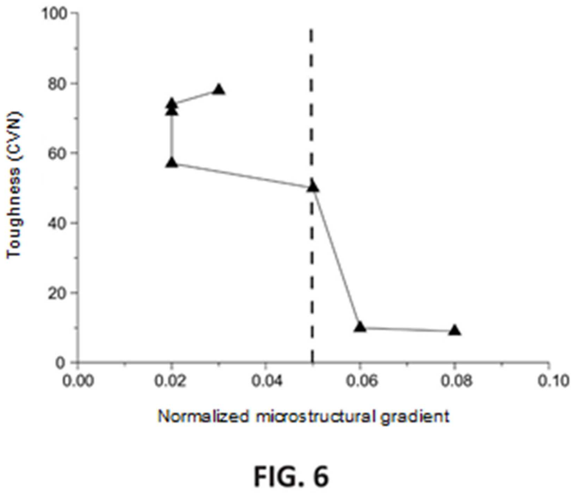

Figure 6.- Shows the toughness value (CVN) at the bond line of tubes with different normalised microstructural gradients in the analysis region obtained according to the method of the present invention.

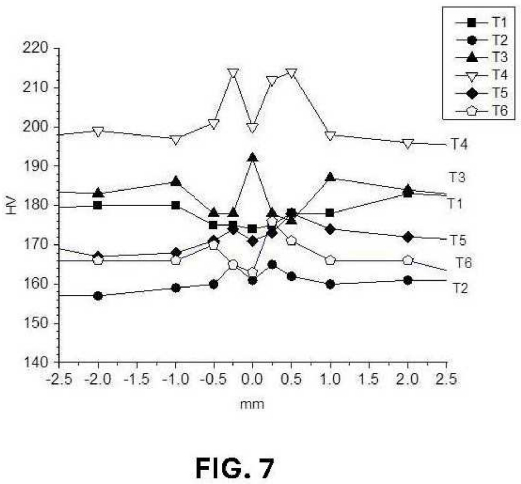

Figure 7.- Shows the Vickers hardness values, HV10, around the bond line for various HFW high frequency induction heating welds.

**PREFERRED EMBODIMENT OF THE INVENTION**

The following is a detailed description of the system for the assessment of the toughness of longitudinal high frequency induction or resistance welds in steel tubes of the present invention, wherein the system comprises:

-   image acquisition means (1) configured to acquire, in use, at least a two-dimensional image (2) of an outer or inner surface of the tube (10) in an analysis region (RA) around a longitudinal bond line (LU), wherein the two-dimensional image (2) comprises a first dimension (2x) essentially perpendicular to the weld bond line and a second dimension (2y) essentially parallel to the bond line (LU), and wherein the two-dimensional image (2) comprises data associated with the microstructure of the tube (10) in the analysis region (RA);

-   processing means (3) configured to provide, from the data associated with the microstructure of the tube (10) in the analysis region (RA), a greyscale profile (4) with respect to the first dimension (2x); and

-   analysis means (5) configured to analyse the greyscale profile (4) and categorise the toughness of the tube (10) in the analysis region (RA) around the bond line (LU) of the longitudinal weld.

Preferably, the system further comprises coupling means (6) of the imaging means (5) to the tube (10), wherein the coupling means (6) comprise a support which in turn comprises a bottom edge with a curvature essentially equal to the curvature of the tube.

EXAMPLE 1

The method of the present invention comprises in a first stage the preparation of the outer or inner surface of the tube. The tubes must be prepared superficially according to the protocol described below:

1.- Automatic grinding using an abrasive disc to remove the outer layers of paint and rust from the pipe down to the metal material. It is necessary to grind an area of at least 2000mm2 with the width and height of this area being similar.

2.- Automatic sanding sequence with sanding discs from coarse to finer grit: 80 - 240 - 320, eliminating the marks of the previous sanding at each stage; and/or

3.- Manual sanding sequence with 400, 600, 1200 and 2000 grit sanding discs, alternating the sanding direction between sanding discs. By means of this sequence, scuff marks and scratches from the previous sequence are eliminated.

4.- Manual polishing with 3 $\mu$m diamond paste applied with a polishing cloth, which leaves a shiny mirror finish.

5.- etching with an alcoholic solution of nitric acid, for a period of time of between 15 and 60 seconds, depending on the chemical composition of the tube studied, where the nitric acid is in a concentration of less than or equal to 5%. The process is carried out manually using cotton wool soaked in the acid. In this stage, it is essential to avoid contact between the acid and the paint on the tube, so as not to soil the area under study.

6.- Cleaning with ethanol and drying of the surface, preferably with forced air (dryer) to remove the ethanol film and not leave marks on the area under study.

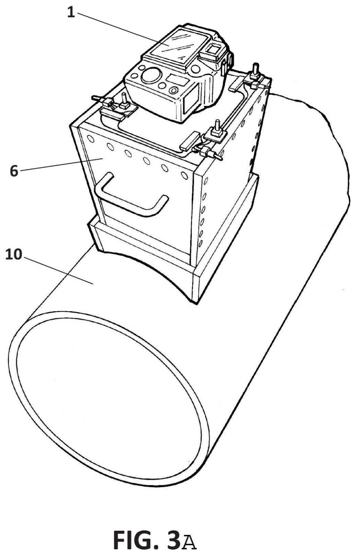

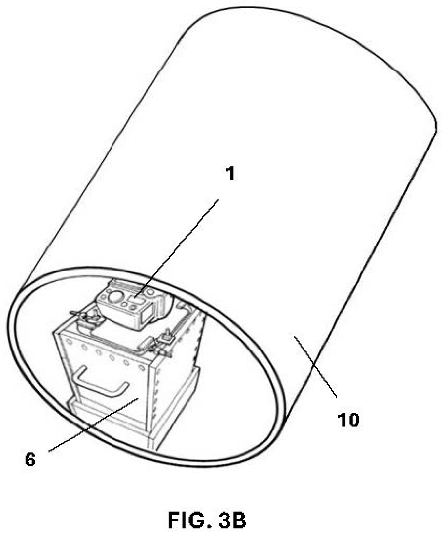

Once the surface has been suitably prepared according to stages 1 to 6, the image acquisition stage proceeds using a device developed to attach to the outer surface of the tube, shown in figure 3.

The images to evaluate the toughness of the bond line (LU) of HFW/ERW tubes are obtained by means of an image acquisition means, preferably a digital camera, coupled to a coupling means or box that is coupled to the outer surface of the tube, made of mechanically resistant material and opaque to the bond line (LU) with an opening that allows the camera to be coupled to take the images.

The images of the bond line (LU) obtained are subsequently evaluated by means of processing means configured to provide, from the data associated with the microstructure of the tube in the analysis region, a greyscale profile with respect to the first dimension, describing the different microstructural zones around the bond line (LU) including the analysis region (RA) that corresponds to the TMAZ. The digital camera allows the adjustment of the different parameters such as bond line (Luminance, focus and ISO) and is configured to acquire a representative image of the area around the bond line (LU) of the weld.

The image acquisition stage is performed as follows:

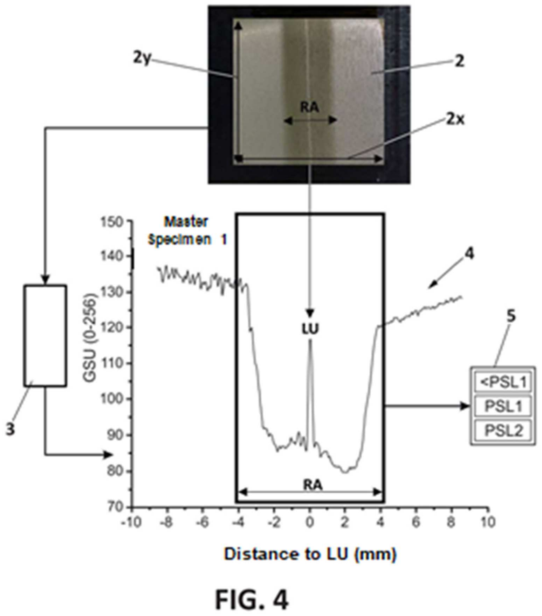

1.- Delimitation of the analysis area of, preferably 25 x 25 mm around the junction line (LU) by applying a black mask that adapts to the surface of the tube and eliminates parasitic reflections. It is necessary to incorporate a sample pattern or scale that allows converting the pixel value of the image to mm, figure 4.

2.- Placement of the image acquisition device. The image acquisition device with the appropriate coupling to the curvature of the tube shall be attached to the area of interest.

3.- Placement of the master specimen in the tube next to the area of interest. Specifically, a first master specimen (Master 1) (A) and a second master specimen (Master 2) (B) will be used as master specimens due to their proven low toughness in the bond line (LU).

The use of master specimen(s) is convenient for several reasons:

a.- to establish an objective comparison of the slightly different illumination conditions that are sometimes necessary to use to get a good image of the sample under study.

b.- to establish a reliable reference for the toughness value of a standardised grey profile in image analysis.

c.- the influence of lighting conditions/sample preparation can be normalised by modifying the grey level of the image using image analysis software and normalised by the average grey level of the profile.

4.- Illumination of the sample. Adequate illumination of the sample is achieved by using a double longitudinal LED light source of at least 10cm in length. This requirement has been found to be necessary to adequately contrast the bond line (LU) with respect to the surrounding area. The power of the LEDs does not necessarily have to be a fixed value because it has to be considered in combination with the parameters exposure time (S), aperture and ISO of the camera when taking the image. That is to say, using a high light power with low S, aperture and ISO parameters is equivalent to using a low light power and high S, aperture and ISO values.

A fundamental aspect in the present invention is to acquire a homogeneous image with respect to its illumination; i.e:

- free of artefacts or reflections.

- independent of natural illumination.

5. Image acquisition of the area of interest (including the master specimens). It is advisable to acquire at least three images at different positions along the bond line (LU) to analyse the reproducibility of the result. Imaging conditions. Focus and exposure time.

Once the sample has been illuminated, focusing of the image for analysis should be performed centred on the bond line (LU). Image analysis shall be performed on the images taken from the tubes using the device without any modification of brightness or contrast, and without applying any filters or modifications to the images.

6.- Analysis of the images obtained. A rectangular area centred on the bond line (LU) up to the edge of the TMAZ is taken for image analysis, as shown in figure 4. The rectangular area should be centred on the bond line (LU) and be wide enough to completely include the analysis region (RA) on both sides of the bond line (LU). The height of the rectangular area should ideally be 10 mm. In this way, the noise of the grey profile is smoothed and the effect of misalignment of the LU on the result is minimised.

If the height of the chosen area were too small, e.g. 3mm, the result would be very noisy. If, on the other hand, it were too large, e.g. 20 mm, there would be a high probability that the misalignment around the bond line (LU) would be distorted.

If the bond line (LU) is not a straight line, the rectangular area must be located so that the upper half of the bond line (LU) is on one side of the axis of the rectangular area and the lower half of the bond line (LU) is on the other side.

Taking into account all the aspects described above, the analysis region (AR) is usually in the range 100-200 in greyscale. Shade 0 corresponds to the colour black and shade 255 to the colour white.

The analysis area has been correctly selected if the obtained greyscale profile reveals the peak corresponding to the bond line (LU) and the change in change in shade of grey between the analysis region (RA) and the adjacent material.

Figures 5a and 5b show the microstructural zones around the bond line (LU) for a sample without normalising treatment, low toughness, master specimen 1 (figure 5a) and one with normalising treatment, high toughness, AT (figure 5b). It can be seen how the different heat treatment generates different shades of grey around the bond line (LU).

The categorisation of the bond line (LU) toughness of the weld is made on the basis of the microstructural gradients described in grey scale around the bond line (LU), figures 5a and 5b, with respect to the grey scale units (GSU) corresponding to the baseline of the bond line (LU). The basis of the present invention is based on the idea that when the microstructural gradient is greater than a given value, the weld has not been performed correctly and the bond line (LU) behaves as a metallurgical notch which greatly decreases the toughness of the bond line (LU). To characterise the metallurgical notch effect, Charpy tests have been performed on samples with different processing and the CVN values have been correlated with the microstructural gradient in the region of the analysis region (RA). This microstructural gradient value, which is identified with the presence of a metallurgical notch, has been calculated as 0.05 from the results obtained and described in figure 6. Figure 6 shows the value of the toughness (CVN) at the bond line (LU) of tubes with different microstructural gradients in the region of analysis (RA).

The evaluation of the bond line (LU) toughness is performed using a scoring system shown in Figure 6 based on the value of the normalised gradient in the region of analysis (RA). The tube is considered to have low toughness if there is a value of T greater than 0.05 being:

$$T = \left(\frac{GSU(RA)}{GSU_0}\right) / widthRA(mm) = 0.05$$

where $GSU_0$ is the grey shade of the base of the peak corresponding to the bond line (LU) and GSU(RA) is the arithmetic difference between the highest and lowest grey shade in the analysis region (RA)

EXAMPLE 2

The method of the present invention comprises in a first stage the preparation of the inner surface of the tube. The surface of the tube must be prepared following the protocol described below:

1.- Laser cleaning by means of a device developed to be coupled to a robotic crawler to remove the layers of paint and rust from the tube until reaching the metallic material. It is necessary to clean an area of at least 2000mm$^2$ being the width and height of this area similar.

2.- Chemically etching with an alcoholic solution of nitric acid by means of a device developed to be coupled to the robotic system, for a period of time of between 15 and 60 seconds, depending on the chemical composition of the tube studied, where the nitric acid is in a concentration of less than or equal to 5%. In this stage, it is essential to avoid contact between the acid solution and the paint on the tube, so as not to soil the area under study.

3.- Cleaning with ethanol and drying of the surface, preferably with forced air (dryer) to drag the ethanol film and not leave marks on the area under study.

Once the surface has been conveniently prepared according to stages 1 to 3, the image acquisition stage is carried out using a device developed to be coupled to the robotic system described above, which moves it along the inside of the tube to analyse the inner surface of the tube, shown in figure 3b.

The images for evaluating the toughness of the bond line (LU) of HFW/ERW tubes are obtained by means of an image acquisition means, preferably a digital camera, coupled to a coupling means mounted on the robotic system. The obtained bond line (LU) images are further evaluated by processing means configured as described in example 1. The digital camera allows the adjustment of the different parameters (Brightness, focus and ISO) and is configured to acquire a representative image of the area around the bond line (LU) of the weld. The image acquisition stage is performed as follows:

1.- Delimitation of the analysis area, preferably 25 x 25 mm2 around the bond line (LU) by applying a black mask that adapts to the surface of the tube and eliminates parasitic reflections.

Positioning of the image acquisition device by displacement of the robotic system. The image acquisition device mounted on the robotic system shall be attached to the area of interest.

3.- Placement of the master specimen in the tube next to the area of interest by means of the robotic system. Specifically, a first master specimen (Master 1) (A) and a second master specimen (Master 2) (B) will be used as master specimens due to their proven low toughness in the bond line (LU). The use of master specimen(s) is convenient for the reasons described in example 1:

4.- Illumination of the sample in the same manner as described in example 1.

5.- Image acquisition of the area of interest (including the master specimen) in the manner described in example 1.

6.- Analysis of the images obtained following the procedure described in example 1. The categorisation of the toughness of the bond line (LU) of the weld is performed in the same way and with the same equation as described in example 1.

EXAMPLE 3

The method of the present invention comprises in a first stage the preparation of the inner surface of the tube. The surface of the tubes must be prepared following the protocol described below:

1.- Automatic grinding using an abrasive disc to remove the outer layers of paint and rust from the tube down to the metallic material. It is necessary to grind an area of at least 2000mm2 being the width and height of this area similar.

2.- Automatic sanding sequence with sanding discs from coarse to finer grit: 80 - 240 - 320, removing the marks of the previous sanding at each stage; and/or it is not always necessary to use all the sanding discs.

3.- Manual sanding sequence with 400, 600, 1200 and 2000 grit sanding papers, alternating the sanding direction between the sanding papers. By means of this sequence, scuff marks and scratches from the previous sequence are eliminated.

4.- Manual polishing with 3 $\mu$m diamond paste applied with a polishing cloth, which leaves a shiny mirror-like finish.

5.- Chemical etching with an alcoholic solution of nitric acid of concentration less than or equal to 5%, for a period of time of 30 seconds, followed by cleaning with alcohol and drying.

6.- Acquisition of the image using a device developed to attach to the outer surface of the tube, shown in figure 3.

7.- Chemical etching with an alcoholic solution of nitric acid of concentration less than or equal to 5%, for a period of time of 15 seconds, followed by cleaning with alcohol and drying.

Acquisition of the image using a device developed to attach to the outer surface of the tube, shown in figure 3a.

9.- Chemical etching with an alcoholic solution of nitric acid of concentration less than or equal to 5%, for a period of 15 seconds, followed by cleaning with alcohol and drying.

Images for assessing the bond line (LU) toughness of HFW/ERW tubes are obtained using the image acquisition means described in figure 3a. The image acquisition stage is performed as described in example 2.

The categorisation of the bond line (LU) toughness of the gas atmosphere weld that embrittles the steel is performed by combining:

i) the calculation of the microstructural gradients described in grey scale around the bond line (LU), figures 5a and 5b, with respect to the grey scale units (GSU) corresponding to the baseline of the bond line (LU). This microstructural gradient value, which is identified with the presence of a metallurgical notch, has been calculated as 0.05 based on the results obtained and described in figure 6 and the equation described in example 1.

ii) The calculation of the hardness around the bond line (LU). The hardness around the bond line (LU) is determined from the profiles obtained from the analysis of the images obtained. The hardness around the bond line (LU) is considered to be proportional to that of the base material, X42, X52, etc, the grain size and the phases present in the area of interest: polygonal ferrite, acicular ferrite, pearlite, bainite, retained austenite, martensite, etc. The maximum recommended hardness to avoid hydrogen embrittlement in pipeline steels is considered as 275 HV.

By means of the imaging technique, we can determine the variations in hardness with respect to the bond line (LU) corresponding in the grey profiles to the central peak. The higher the hardness (finer the grain and finer the pearlite/bainite/martensite), the faster the nital etching.

The procedure consists of etching with nital around the bond line (LU) for several times and then cleaning with alcohol and drying, making an image acquisition after each of the etching, cleaning and drying processes. An image of the area of interest is acquired and the descriptive profile of the area of interest is calculated. The process is repeated with a longer etching time, for example an additional 30 seconds, followed by cleaning and drying and images are acquired again and the corresponding profile is calculated. The process is repeated a third time (without prejudice to repeating the process as many times as necessary) with a longer etching time, e.g. an additional 30 seconds, and an image is re-acquired and the corresponding profile is calculated. In this case the total etching time with nital would be 90 seconds.

The difference in grey shade between the three profiles is then calculated for the 30, 60 and 90 second etching times. The GSU grey values decrease with etching time as a function of the microstructure around the bond line (LU).

The determination of the hardness starts by assigning a HV value to the base material which can be obtained from the yield strength requirement of the base material according to the standards. The following hardness values are considered for the base material: 160HV for X42, 170HV for Grade B. and 190HV for X60.

The hardness value around the LU increases with respect to the base material if the microstructure is fine-grained with a perlitic/bainitic structure. This type of microstructure is evident in a grey shade profile in which the bond line (LU) (central peak of the profile) is rapidly revealed (< 30s) and this profile is maintained for consecutive etching s up to high etching times, e.g. 90 seconds. However, if the microstructure around the bond line (LU) is coarse-grained pearlitic, a decrease in hardness occurs. In this coarse microstructure it is typical that no decrease in grey shades (GSU) occurs until high etching times. There is a relationship between hardness and GSU. In Grade B and X42 materials the ratio is inversely proportional to 1HV:3GSU. In the X60 material the ratio is 1HV:1GSU. The decrease in hardness is inversely proportional to the decrease in GSU.

The hardness predictions made according to the method described for the curves summarised in figure 7 are shown below.

T1: When the nital etching is performed, there is a progressive drop of 18 GSU up to 30s indicating that the microstructure is medium (neither fine nor coarse). There is a 1HV:3 GSU equivalence in X42 materials such as T1, therefore the hardness should increase by 6HV around the bond line (LU) from 160HV to 166HV.

T2: On etching with nital, there is a progressive drop of 17 GSU up to 45s indicating a fine grain and coarse pearlitic structure. There is a 1HV:3 GSU equivalence, therefore the hardness should increase by 5HV around the bond line (LU) from 160 to 165HV.

T3: When performing the nital etching, there is no GSU drop until 45s which drops 13GSU. There is a 1HV:1GSU equivalence in X60 materials, so the hardness should drop 13HV around the LU from 190 to 177HV.

T4: 20GSU drop in 15s in the nital etching to keep the profile the same until 45s. There is a 1HV:1GSU equivalence in X60 materials, so the hardness should increase 20HV around the LU from 190 to 210HV.

T5: The weld is slightly asymmetrical. When etching with nital, there is a drop of 15GSU on the sides of the LU from 220GSU in just 15s which is maintained until 45s. There is a GSU to HV equivalence of 1HV:3GSU typical of X42/Grade B materials such as T5, therefore, hardness should increase by 5HV around the bond line (LU) from 170 to 175HV.

T6: The weld is asymmetrical. When the nital etching is performed, there is a drop of 5 GSU on the sides of the bond line (LU) in only 15s which is maintained until 45s. There is an equivalence of 1HV:3 GSU in materials such as T6. The hardness has to increase 1-2 HV around the LU from 170 to 172HV.

**Claims**

1. A system for assessing the toughness of longitudinal high frequency induction or resistance welds in steel tubes, comprising:

   - image acquisition means (1) configured to acquire, in use, at least a two-dimensional image (2) of an outer or inner surface of the tube (10) in an analysis region (RA) around a bond line (LU) of the longitudinal weld, wherein the two-dimensional image (2) comprises a first dimension (2x) essentially perpendicular to the bond line of the weld and a second dimension (2y) essentially parallel to the bond line (LU), and wherein the two-dimensional image (2) comprises data associated with the microstructure of the tube (10) in the analysis region (RA);
   - processing means (3) configured to provide, from the data associated with the microstructure of the tube (10) in the analysis region (RA), a greyscale profile (4) with respect to the first dimension (2x); and
   - analysis means (5) configured to analyse the greyscale profile (4) and categorise the toughness of the tube (10) in the analysis region (RA) around the bond line (LU) of the longitudinal weld.

2. System according to claim 1, wherein the analysis region preferably corresponds to a thermo-mechanically affected zone (TMAZ).

3. System according to any one of the preceding claims, further comprising coupling means (6) of the image acquisition means (5) to the tube (10), wherein the coupling means (6) comprise a base further comprising a bottom edge with a curvature essentially equal to the curvature of the tube (10).

4. System according to claim 3, wherein the coupling means (6) comprise anchoring means configured to attach the imaging means to the surface of the tube, preferably magnetic or electromagnetic anchors.

5. A system according to any one of the preceding claims, further comprising automatic grinding means configured, in use, to remove the outer or inner layers of the tube down to the metallic material.

6. System according to claim 5, further comprising sanding means configured, in use, for sanding the metallic material of the tube.

7. System according to claim 6, further comprising polishing means configured, in use, to obtain a mirror-like glossy finish on the tube.

8. System according to claim 7, further comprising chemical means configured, in use, to chemically etch the tube.

9. System according to claim 8, further comprising cleaning means configured, in use, to remove the film left on the tube by the chemical means so as not to leave marks in the analysis region.

10. System according to claim 9, the system further comprising a robotic module configured to move the surface preparation means and/or the image acquisition means (1) along the tube.

11. A method for the assessment of the toughness of longitudinal high frequency induction or resistance welds in steel tubes, comprising:

- an image acquisition stage wherein at least one two-dimensional image of an outer or inner surface of the tube is acquired in an analysis region around a bond line (LU) of the longitudinal weld, wherein the acquisition stage is carried out such that the two-dimensional image comprises a first dimension essentially perpendicular to the bond line of the weld and a second dimension essentially parallel to the bond line, and wherein in the acquisition stage data associated with the microstructure of the tube in the analysis region (RA) are obtained from the two-dimensional image;
- a processing stage where a greyscale profile with respect to the first dimension is provided from the data associated with the microstructure of the tube in the analysis region (RA); and
- an analysis stage where the greyscale profile is analysed and the toughness of the tube in the analysis region around the weld bond line is categorised.

12. Method according to claim 11, further comprising a tube coupling stage prior to the image acquisition stage.

13. A method according to any one of claims 11 or 12, further comprising an automatic grinding stage wherein the outer or inner layers of the tube are removed down to the metallic material.

14. Method according to claim 13, wherein the automatic grinding stage uses an abrasive disc to remove the outer layers of the tube down to the metallic material, roughing an area of at least 2000mm$^2$.

15. The method according to any one of claims 13 or 14, further comprising a sanding stage wherein the sanding of the metal material of the tube is carried out.

16. Method according to claim 15, wherein the sanding stage comprises a first automatic sanding sequence with sanding disc from coarse to finer grit: 80 - 240 - 320, removing at each stage the marks of the previous sanding, and/or a manual sanding sequence with 400, 600, 1200 and 2000 grit sanding papers alternating the sanding direction between sanding papers.

17. A method according to any one of claims 15 or 16, further comprising a stage of polishing the tube to obtain a mirror-like bright finish on the tube.

18. Method according to claim 17, wherein the polishing stage is a manual polishing stage with 3 $\mu$m diamond paste applied with a polishing cloth.

19. Method according to any one of claim 17 or 18, further comprising a chemical etching stage of the tube.

20. Method according to claim 19, wherein the chemical etching stage of the tube is carried out with an alcoholic solution of nitric acid, for a period of time of between 15 and 60 seconds, wherein the nitric acid is at a concentration of less than or equal to 5%.

21. Method according to claim 19, wherein the chemical etching stage of the tube is carried out with an alcoholic solution of nitric acid, for a period of time of 15 seconds and a first image is acquired, then another one of 30 seconds and a second image is acquired and finally another one of 45 seconds, wherein the nitric acid is in a concentration less than or equal to 5%.

22. Method according to claim 21, wherein the hardness around the bond line (LU) is determined by the equivalence between GSU and HV from the evolution of the grey profiles for the different etching times.

23. Method according to any one of claim 19 or 20, further comprising a cleaning stage wherein the film left on the tube after the chemical etching stage is removed.

FIG. 1

**FIG. 2A**

**FIG. 2B**

**FIG. 3**A

**FIG. 3B**

FIG. 4

**FIG. 5A**

**FIG. 5B**

**FIG. 6**

**FIG. 7**

## INTERNATIONAL SEARCH REPORT

| International application No |
| --- |
| PCT/ES2024/070297 |

**A. CLASSIFICATION OF SUBJECT MATTER**

INV. G01N21/952    G01N21/954    B23K31/12    B65B51/22    G01N1/28
G01N1/32    G01N1/34    G06T7/00

ADD.

According to International Patent Classification (IPC) or to both national classification and IPC

**B. FIELDS SEARCHED**

Minimum documentation searched (classification system followed by classification symbols)

G01N    G06T    B65B    B23K

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
| --- | --- | --- |
| X | RAVIKIRAN KOPPARTHI ET AL: "Microstructure and crystallographic texture of high frequency electric resistance welded X65 pipeline steel", MATERIALS CHEMISTRY AND PHYSICS, ELSEVIER SA, SWITZERLAND, TAIWAN, REPUBLIC OF CHINA, vol. 302, 13 April 2023 (2023-04-13), XP087302252, ISSN: 0254-0584, DOI: 10.1016/J.MATCHEMPHYS.2023.127758 [retrieved on 2023-04-13] | 1,2,5-9, 11,13-23 |
| Y | abstract; figures 1,6,7[a] page 2, right-hand column, last paragraph - page 3, left-hand column, paragraph 1 page 4, right-hand column, paragraph 4 page 7, left-hand column, paragraph 2 ----- -/-- | 3,4,10, 12 |

| [x] Further documents are listed in the continuation of Box C. | [x] See patent family annex. |
| --- | --- |

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 31 July 2024 | 19/08/2024 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Duijs, Eric |
| --- | --- |

Form PCT/ISA/210 (second sheet) (April 2005)

**EP 4 715 375 A1**

## INTERNATIONAL SEARCH REPORT

International application No

PCT/ES2024/070297

**C(Continuation).  DOCUMENTS CONSIDERED TO BE RELEVANT**

| Category* | Citation of document, with indication, where appropriate, of the relevant passages | Relevant to claim No. |
|---|---|---|
| X | US 2005/041852 A1 (SCHWARZ JOACHIM [DE] ET AL) 24 February 2005 (2005-02-24) | 1,11 |
| Y | paragraphs [0001], [0003], [0007] - [0010], [0022] - [0024]; figures 1-4 | 3,4,10, 12 |
| | - - - - - | |
| Y | US 2016/320266 A1 (KIMPEL JR RICK R [US] ET AL) 3 November 2016 (2016-11-03) paragraphs [0002], [0004], [0023], [0024], [0027], [0028], [0040]; figures 1,3 | 3,4,12 |
| | - - - - - | |
| A | CN 110 514 148 A (CHINA NAT PETROLEUM CORP; CNPC TUBULAR GOODS RES INSTITUTE) 29 November 2019 (2019-11-29) claims 1,3; figures 1,2 | 6,9,15, 16,23 |
| | - - - - - | |
| Y | CN 218 657 472 U (SHANGHAI MORIMATSU PHARMACEUTICAL EQUIPMENT ENG CO LTD) 21 March 2023 (2023-03-21) claim 1; figure 1 | 10 |
| | - - - - - | |

Form PCT/ISA/210 (continuation of second sheet) (April 2005)

## INTERNATIONAL SEARCH REPORT
### Information on patent family members

| | International application No |
|---|---|
| | PCT/ES2024/070297 |

| Patent document cited in search report | | | Publication date | Patent family member(s) | | Publication date |
|---|---|---|---|---|---|---|
| US 2005041852 | A1 | | 24-02-2005 | AT | E506138 T1 | 15-05-2011 |
| | | | | CA | 2465231 A1 | 22-05-2003 |
| | | | | EP | 1448334 A1 | 25-08-2004 |
| | | | | ES | 2363078 T3 | 20-07-2011 |
| | | | | JP | 4531396 B2 | 25-08-2010 |
| | | | | JP | 2005508759 A | 07-04-2005 |
| | | | | KR | 20050044429 A | 12-05-2005 |
| | | | | PT | 1448334 E | 28-06-2011 |
| | | | | US | 2005041852 A1 | 24-02-2005 |
| | | | | WO | 03041902 A1 | 22-05-2003 |
| US 2016320266 | A1 | | 03-11-2016 | CA | 2906711 A1 | 18-09-2014 |
| | | | | CA | 2992772 A1 | 18-09-2014 |
| | | | | GB | 2527988 A | 06-01-2016 |
| | | | | MX | 349327 B | 21-07-2017 |
| | | | | US | 2014260705 A1 | 18-09-2014 |
| | | | | US | 2016320266 A1 | 03-11-2016 |
| | | | | WO | 2014144382 A2 | 18-09-2014 |
| CN 110514148 | A | | 29-11-2019 | NONE | | |
| CN 218657472 | U | | 21-03-2023 | NONE | | |

Form PCT/ISA/210 (patent family annex) (April 2005)

# EP 4 715 375 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Nondestructive Evaluation for Yield Strength and Toughness of Steel Pipelines. **S. D. PALKOVIC** ; **K. TANIGUCHI** ; **S. C. BELLEMARE**. Massachusetts Materials Technologies, LLC, Cambridge, MA 2. Massachusetts Institute of Technology, 2018